# EUROPÄISCHE PATENTSCHRIFT

(11) **EP 3 353 424 B1**
(45) Veröffentlichungstag und Bekanntmachung des Hinweises auf die Patenterteilung: **25.03.2020**
(21) Anmeldenummer: 16782011.7
(22) Anmeldetag: 21.09.2016
(51) Int. Cl.: F04D 17/16, F04D 25/06, F04D 25/08, F04D 29/08, F04D 29/58, F04D 29/66

(54) **LÜFTEREINHEIT**
FAN UNIT
ENSEMBLE VENTILATEUR

(30) Priorität: 24.09.2015 DE 102015012277
(43) Veröffentlichungstag der Anmeldung: 01.08.2018
(73) Patentinhaber: Ebm-Papst St. Georgen GmbH & CO. KG, 78112 St. Georgen (DE)
(72) Erfinder: RAGG, Peter, 78141 Schönwald (DE)
(74) Vertreter: Staeger & Sperling Partnerschaftsgesellschaft mbB
(86) Internationale Anmeldenummer: PCT/EP2016/001574
(87) Internationale Veröffentlichungsnummer: WO 2017/050427

(56) Entgegenhaltungen:
- EP-A2- 1 688 622
- US-A- 2 316 608
- US-A- 3 199 774
- US-A1- 2010 189 554
- US-A1- 2014 014 109

## Beschreibung

Die vorliegende Erfindung betrifft eine Lüftereinheit mit einem Gehäuse, einem in einer Radkammer des Gehäuses untergebrachten Lüfterrad und einem mit dem Lüfterrad über eine Welle verbundenen, in einer Motorkammer des Gehäuses untergebrachten Motor.

Aus US 8 267 674 B2 ist eine solche Lüftereinheit bekannt, bei der zur Kühlung des Motors Durchgänge zwischen Radkammer und Motorkammer so angeordnet sind, dass die Drehung des Lüfterrades auch einen Luftstrom durch die Motorkammer antreibt.

Die Kühlluft wird bei dieser bekannten Lüftereinheit unmittelbar durch den Motor hindurchgeführt, was zwar eine effiziente Kühlung ermöglicht, gleichzeitig aber das Risiko mit sich bringt, dass die Luft beim Durchgang durch den Motor im Kontakt mit im Betrieb erhitzten Wicklungen und mit schmierstoffbedeckten Oberflächen Gerüche aufnimmt, die sich in der Radkammer unter die umgewälzte Luft mischen und im Blasluftstrom der Lüftereinheit noch wahrnehmbar sind.

Der Motor ist in der Motorkammer durch unmittelbaren Kontakt mit deren Wänden fixiert. Schwingungen des Motors können sich daher ungedämpft auf die Wände des Gehäuses übertragen und diese zur Geräuschemission anregen.

Bei einer Lüftereinheit, die, wie in US 8 267 674 B2 angegeben, für KFZ-Anwendungen vorgesehen ist, fällt das Betriebsgeräusch der Lüftereinheit gegenüber der eines gleichzeitig betriebenen Verbrennungsmotors nicht ins Gewicht, und auch eine eventuelle Geruchsbelastung wird gegenüber anderen, stärkeren Gerüchen des Stra-ßenverkehrs kaum wahrgenommen. Bei einer Lüftereinheit, die für medizinische Anwendungen wie etwa ein Gerät zu Atmungsunterstützung vorgesehen ist, sind ein geräuscharmer Betrieb und die Freiheit der Blasluft von unerwünschten Gerüchen von ungleich höherer Bedeutung.

Weiterer Stand der Technik aus dem vorliegenden technischen Gebiet ist aus den Schriften US 2 316 608 A, US 2014/014109 A1, US 3 199 774 A und EP 1 688 622 A2 bekannt.

Aufgabe der Erfindung ist daher, eine Lüftereinheit zu schaffen, die sowohl einen geräuscharmen Betrieb ermöglicht als auch eine Geruchsbelastung der Blasluft wirksam unterbindet.

Die Aufgabe wird durch eine Lüftereinheit gemäß den Merkmalen des Anspruchs 1 gelöst, indem bei der Lüftereinheit mit einem Gehäuse, einem in einer Radkammer des Gehäuses untergebrachten Lüfterrad und einem mit dem Lüfterrad über eine Welle verbundenen, in einer Motorkammer des Gehäuses untergebrachten Motor der Motor zwischen Wänden der Motorkammer durch Puffer fixiert ist, wobei wenigstens einer der Puffer ein Dichtring ist, der die Welle von einem sich in einem radialen Schnitt ringförmig um den Motor erstreckenden Kühlluftkanal trennt. Die Fixierung durch Puffer an zwei Seiten des Motors erlaubt es, einen direkten Kontakt des Motors mit den Wänden des Gehäuses zu vermeiden und es somit eine Übertragung von Schwingungen des Motors auf das Gehäuse allenfalls in durch die Puffer gedämpfter Form zuzulassen, was die Geräuschemission über das Gehäuse merklich reduziert. Indem wenigstens einer der Puffer als Dichtring ausgebildet ist, verhindert er die Ausbreitung von Gerüchen, die insbesondere von einem Lager der Welle im Motor ausgehen können, in die durch den Kühlluftkanal zirkulierende Luft.

Der Innendurchmesser des Dichtrings sollte hierfür größer sein als der Außendurchmesser eines Spalts zwischen gegeneinander rotierenden Teilen des Lagers.

Die Puffer können eigenständige Bauteile sein, die wie der Motor bei der Montage der Lüftereinheit in das Gehäuse eingefügt werden. Vorzugsweise sind sie, um die Montage der Lüftereinheit zu vereinfachen, mit dem Gehäuse durch Mehrkomponenten-Spritzguss zu einer Baueinheit verbunden.

Die Wände, zwischen denen der Motor durch die Puffer gehalten ist, sind vorzugsweise in Richtung der Welle beabstandet; dies vereinfacht den Zusammenbau der Lüftereinheit.

Wenn nicht nur einer, sondern zwei Dichtringe vorgesehen sind, kann der Kühlluftkanal durch die Dichtringe, eine sich zwischen den Dichtringen erstreckende Außenfläche des Motors und eine sich zwischen den Dichtringen erstreckende Innenfläche des Gehäuses begrenzt sein.

Die an den Kühlluftkanal angrenzende Außenfläche des Motors ist vorzugsweise durch ein Statorpaket gebildet. Über dessen Metall kann Wärme von den Wicklungen des Motors effizient abgeleitet und auf die Luft im Kühlluftkanal übertragen werden, ohne dass die Gefahr einer Geruchsbelastung besteht. Wenn das Statorpaket nicht freiliegt, sondern in ein Motorgehäuse eingeschlossen ist, dann sollte dessen Wand zumindest direkt an dem Statorpaket anliegen, um den Wärmeabfluss zum Kühlluftkanal zu erleichtern.

Um eine effiziente Kühlung auch bei geringem Luftdurchsatz des Kühlluftkanals zu gewährleisten, ist erfindungsgemäß vorgesehen, dass der ringförmige Kühlluftkanal an einer Stelle von einer Trennwand unterbrochen ist und dass ein Einlass und ein Auslass des Kühlluftkanals benachbart zur Trennwand auf verschiedenen Seiten derselben angeordnet sind, um die Kühlluft dazu zu zwingen, in Umfangsrichtung um den Motor herum zu strömen und so dessen Wärme aufzunehmen.

Von Einlass und Auslass des Kühlluftkanals sollte wenigstens einer mit der Radkammer verbunden sein.

Wenn insbesondere der Einlass des Kühlluftkanals von einem sich rund um das Lüfterrad erstreckenden Blasluftkanal abzweigt, kann ein in dem Blasluftkanal herrschender Überdruck genutzt werden, um die Zirkulation durch den Kühlluftkanal anzutreiben.

Der Auslass des Kühlluftkanals sollte seinerseits vorzugsweise ebenfalls wieder in die Radkammer einmünden, damit die im Kühlluftkanal fließende Luft dem Blasluftstrom der Lüftereinheit nicht verloren geht.

Der Luftdurchsatz des Blasluftkanals nimmt von einem inneren Ende zu einem äußeren Ende, an dem sich eine Ausblasöffnung für den Blasluftstrom befindet, kontinuierlich zu. Um eine zwischen den Enden des Blasluftkanals bestehende Druckdifferenz zum Antreiben des Kühlluftstroms auszunutzen, sollte von Ein- und Auslass des Kühlluftkanals der eine benachbart zum inneren Ende und der andere benachbart zum äußeren Ende von dem Blasluftkanal abzweigen.

Das Gehäuse der Lüftereinheit ist im Allgemeinen aus mehreren Teilen zusammengesetzt. Insbesondere können ein den Motor aufnehmendes erstes Gehäuseteil und ein zweites Gehäuseteil, das eine Ansaugöffnung umfasst, in einer Außenwand des Blasluftkanals aneinandergrenzen.

Erstes und zweites Gehäuseteil sind vorzugsweise durch Rasthaken miteinander verbunden.

Eine Zwischenwand zwischen Motor- und Radkammer ist vorzugsweise am ersten Gehäuseteil befestigt, insbesondere verschraubt. Ein Vorteil dieses Aufbaus liegt zum einen darin, dass, da die Zwischenwand innerhalb des Gehäuses eingeschlossen ist, entlang ihrer Ränder keine Blasluft aus dem Gehäuse verlorengehen kann und die Zwischenwand deshalb an ihren Rändern nicht dicht an das erste Gehäuseteil anzuschließen braucht, um Luftverluste zu verhindern. Indem die Befestigung durch Verschrauben erfolgt, kann darüber hinaus die Klemmung des Motors zwischen den Puffern eingestellt werden.

Eine Stiftleiste, die wenigstens für die Stromversorgung des Motors dient, eventuell aber auch für die Übertragung von Steuersignalen zum Motor oder von Sensorsignalen zu einer externen Steuereinheit dienen kann, ist vorzugsweise dicht in eine Wand des Gehäuses eingespritzt.

Weitere Merkmale und Vorteile der Erfindung, deren Schutzumfang ausschließlich durch die nachfolgenden Ansprüche bestimmt ist, ergeben sich aus der nachfolgenden Beschreibung von Ausführungsbeispielen unter Bezugnahme auf die beigefügten Figuren. Es zeigen:
- Fig. 1: einen radialen Schnitt durch eine erfindungsgemäße Lüftereinheit entlang einer in Fig. 2 und 3 jeweils mit I-I bezeichneten Ebene;
- Fig. 2: einen axialen Schnitt durch die Lüftereinheit entlang einer in Fig. 1 mit II-II bezeichneten Ebene;
- Fig. 3: einen axialen Schnitt entlang einer zur Ebene II-II orthogonalen, in Fig. 1 mit III-III bezeichneten Ebene;
- Fig. 4: einen axialen Schnitt durch die Lüftereinheit entlang einer in Fig. 3 mit IV-IV bezeichneten Ebene;
- Fig. 5: einen axialen Schnitt durch die Lüftereinheit entlang einer in Fig. 3 mit V-V bezeichneten Ebene;
- Fig. 6: eine Draufsicht auf die Lüftereinheit;
- Fig. 7: eine Seitenansicht;
- Fig. 8: eine Ansicht der Lüftereinheit von unten; und
- Fig. 9: eine Stiftleiste der Lüftereinheit.

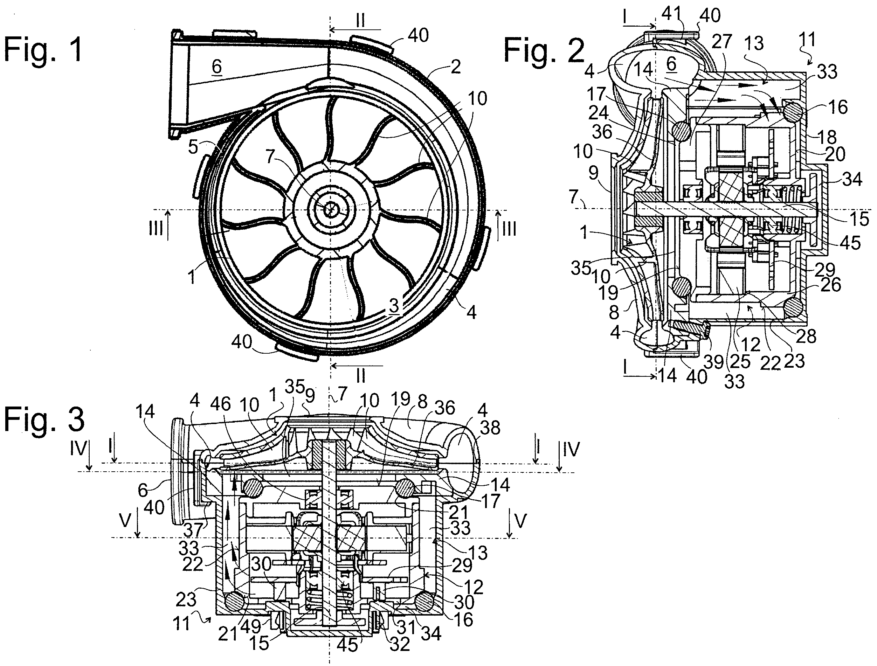

Fig. 1 zeigt einen radialen Schnitt durch die erfindungsgemäße Lüftereinheit in Höhe eines Schaufelrades 1. In einem aus mehreren Spritzformteilen aus Kunststoff zusammengefügten Gehäuse 2 ist eine Radkammer 3 gebildet, von der das Schaufelrad 1 einen zentralen Bereich einnimmt. Rings um den zentralen Bereich erstreckt sich ein Blasluftkanal 4, dessen Querschnittsabmessungen bezogen auf eine Drehachse 7 des Schaufelrads 1 sowohl in radialer als auch in axialer Richtung von einem Anfangspunkt 5 im Gegenuhrzeigersinn bis zu einem Endpunkt an einer Ausblasöffnung 6 kontinuierlich zunehmen.

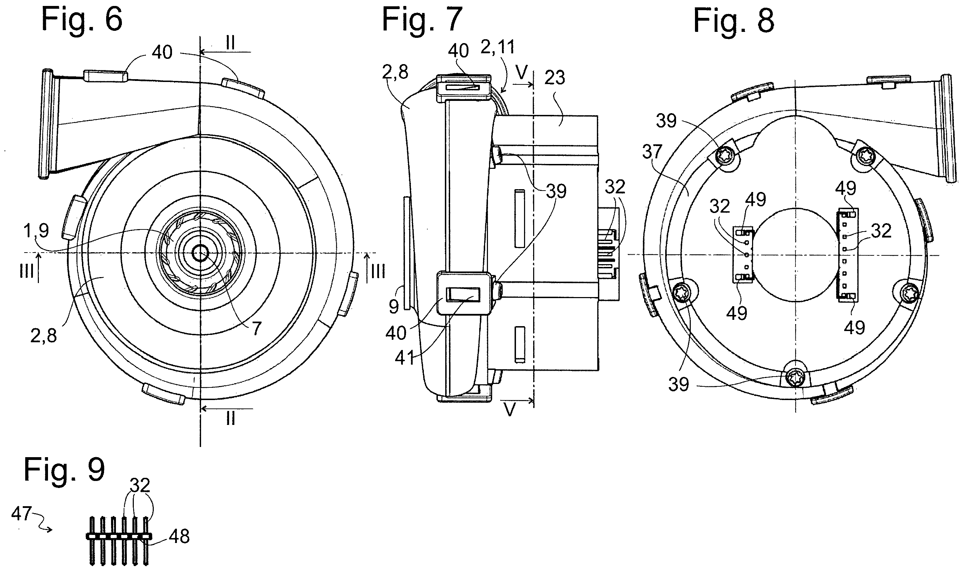

Wie in den Schnitten der Fig. 2 und 3 und der Draufsicht der Fig. 6 zu erkennen, ist in einem Oberteil 8 des Gehäuses 2 in Verlängerung der Drehachse 7 des Schaufelrades 1 eine Ansaugöffnung 9 gebildet. Luft, die über die Ansaugöffnung 9 in das Gehäuse 2 eintritt, wird von den im Gegenuhrzeigersinn rotierenden Schaufeln 10 des Schaufelrades 1 radial nach außen beschleunigt und im Blasluftkanal 4 in Richtung der Ausblasöffnung 6 getrieben.

In einem becherförmigen Unterteil 11 des Gehäuses 2 ist eine Motorkammer 13 geformt, in der ein Elektromotor 12 untergebracht ist. Eine als von Oberteil 8 und Unterteil 11 separates Gehäusebauteil ausgebildete Zwischenwand 14 trennt die Motorkammer 13 von der Radkammer 3. Eine Welle 15 des Elektromotors 12 ragt durch eine Öffnung der Zwischenwand 14 in die Radkammer 3 hinein, um das Schaufelrad 1 zu tragen.

Der Motor 12 hat einen im Wesentlichen zylindrischen Körper und ist in der Motorkammer 13 mithilfe von zwei elastischen Dichtringen 16, 17 fixiert, die jeweils zwischen Stirnflächen 19, 20 des zylindrischen Körpers und einer Bodenplatte 18 des Gehäuseunterteils 11 bzw. der Zwischenwand 14 geklemmt sind. Im hier gezeigten Fall fixieren die Dichtringe 16, 17 den Motor 12 gleichzeitig auch in radialer Richtung, indem sie jeweils am Motor 12 in eine Hohlkehle 21 am Übergang zwischen der Stirnfläche 19 bzw. 20 und einer Umfangsfläche 22 des zylindrischen Körpers eingreifen und sich andererseits an einer rings um die Bodenplatte 18 umlaufenden Wand 23 des Unterteils 11 bzw. an der Innenseite einer zum Motor 12 hin offenen Aussparung 24 der Zwischenwand 14 abstützen.

Die Dichtringe 16, 17 können vom Unterteil 11 bzw. der Zwischenwand 14 getrennte Bauteile sein, die beim Zusammenbau der Lüftereinheit angefügt werden. Bevorzugtermaßen sind der Dichtring 16 und das Unterteil 11 bzw. der Dichtring 17 und die Zwischenwand 14 schon vor dem Zusammenbau verbunden, indem durch Mehrkomponenten-Spritzguss der Dichtring an das Unterteil 11 oder die Zwischenwand angeformt wird, oder indem umgekehrt ein Dichtring in die Spritzform des Unterteils 11 oder der Zwischenwand eingelegt und letztere an den Dichtring angeformt wird.

Die Umfangsfläche 22 könnte unmittelbar durch ein Statorpaket des Motors gebildet sein; im hier betrachteten Fall ist das Statorpaket 25 in ein Gehäuse eingeschlossen, das aus zwei ineinandergreifenden Schalenteilen, einem Becher 26 und einem Deckel 27, zusammengefügt ist, von denen der eine die Stirnfläche 20 und der andere die Stirnfläche 19 bildet, und die Umfangsfläche 22 ist im Wesentlichen von der Außenseite einer umlaufenden Wand des Bechers 26 gebildet. An der Innenseite dieser umlaufenden Wand ist eine Schulter 28 geformt. Zwischen Schulter 28 und Boden des Bechers 26 ist eine Elektronikplatine 29 untergebracht, die über Steckbuchsen 30 (s. Fig. 3) mit Energie versorgt wird. Den Steckbuchsen 30 liegen jeweils Öffnungen 31 in der Stirnfläche 20 gegenüber, so dass in der Bodenplatte 18 des Gehäuseunterteils 11 verankerte Kontaktstifte 32 in die Steckbuchsen 30 der Elektronikplatine 29 eingreifen können, ohne das Gehäuse des Motors 12 zu berühren. Schwingungen, zu denen der Becher 26 durch die Drehbewegung angeregt wird, werden daher über die Kontaktstifte 32 allenfalls in durch die Elektronikplatine 29 stark gedämpfter Form an das Gehäuseunterteil 11 übertragen.

Die Verankerung der Kontaktstifte 32 in der Bodenplatte 18 wird erreicht, indem eine Stiftleiste 47 von an sich bekannter Art (siehe Fig. 9), bei der die Kontaktstifte 32 in einem schmalen Kunststoffstab 48 eingebettet sind, in einer Spritzform montiert und das Gehäuseunterteil 11 an den Stab 48 angespritzt wird. Vorzugsweise ist der Kunststoff, aus dem das Gehäuseunterteil 11 gespritzt wird, derselbe wie der des Stabs 48, so dass letzterer beim Spritzen mit der Bodenplatte 18 einsteilig verschmilzt. Außen an die Bodenplatte 18 benachbart zu den Kontaktstiften 32 angeformte Rasthaken 49 (siehe auch Fig. 8) dienen zur formschlüssigen Verankerung eines (nicht dargestellten) Steckers eines Versorgungskabels.

Das Statorpaket 25 ist in den Becher 26 bis zur Schulter 28 eingeschoben und steht in großflächigem reibschlüssigem Kontakt mit der Innenseite der umlaufenden Wand, so dass Abwärme des Statorpakets 25 zur Umfangsfläche 22 abgeführt wird.

Die Dichtringe 16, 17 unterteilen die Motorkammer 13 in einen Kühlluftkanal 33, der sich zwischen der Umfangsfläche 22 des Motors 12 und der Wand 23 des Gehäuseunterteils 11 erstreckt und in axialer Richtung durch die Dichtringe 16, 17 begrenzt ist, einen Raum 34 zwischen der Bodenplatte 18 und der gegenüberliegenden Stirnfläche 20 des Motors 12 sowie einen Raum 35 zwischen der Stirnfläche 19 des Motors 12 und der Zwischenwand 14. Im hier betrachteten Fall weist die Zwischenwand 14 eine geräumige Öffnung auf, über die der Raum 35 mit einem durch eine Bodenplatte 36 des Schaufelrads 1 begrenzten unteren Bereich der Radkammer 3 zusammenhängt.

Wie am Besten in Fig. 3 zu erkennen, ist das Unterteil 11 entlang der Oberkante der Wand 23 radial zu einem Flansch 37 aufgeweitet, um eine die Zwischenwand 14 rings umgreifende Aussparung sowie, oberhalb der Zwischenwand 14, einen unteren Teil der Radkammer 2 zu bilden. Der Blasluftkanal 4 ist in seiner unteren Hälfte teils von der Zwischenwand 14, teils von dem Gehäuseunterteil 11 begrenzt. Entlang der Ebene I-I treffen Ränder des Gehäuseober- und Unterteils 8 bzw. 11 aufeinander, so dass die Zwischenwand 14 vollständig innerhalb des Gehäuses 2 verborgen ist. Die Ränder haben zueinander komplementäre gestufte Konturen, die im Wesentlichen luftdicht ineinander eingreifen.

Zur Befestigung der Zwischenwand dienen mehrere Schrauben 39 (siehe auch Fig. 7, 8), die, wie in Fig. 2 gezeigt, in ungefähr axialer Richtung durch den Flansch 37 hindurch in ein Gewinde der Zwischenwand 14 eingreifen, so dass durch Anziehen der Schrauben 39 die Dichtringe 16, 17 zwischen dem Motor 12 und der Zwischenwand 14 bzw. dem Motor 12 und der Bodenplatte 18 in axialer Richtung komprimiert werden.

So ist der Motor 12 in der Motorkammer 13 allein durch die Dichtringe 16, 17 in einer Stellung fixiert, in der kein direkter Kontakt zwischen dem Motor 12 und den Wänden 23, 18 der Motorkammer 13 besteht. Schwingungen, die der Motor im Betrieb erzeugt, werden daher im Wesentlichen nur über die Dichtringe 16, 17 an das Gehäuse übertragen und dabei gedämpft.

Ober- und Unterteil 8, 11 des Gehäuses 2 sind durch Verrastung zusammengehalten, hier mithilfe von elastischen biegsamen Rasthaken 40 des Oberteils 8, die auf Rastnasen 41 des Gehäuseunterteils 11 aufgeschoben sind.

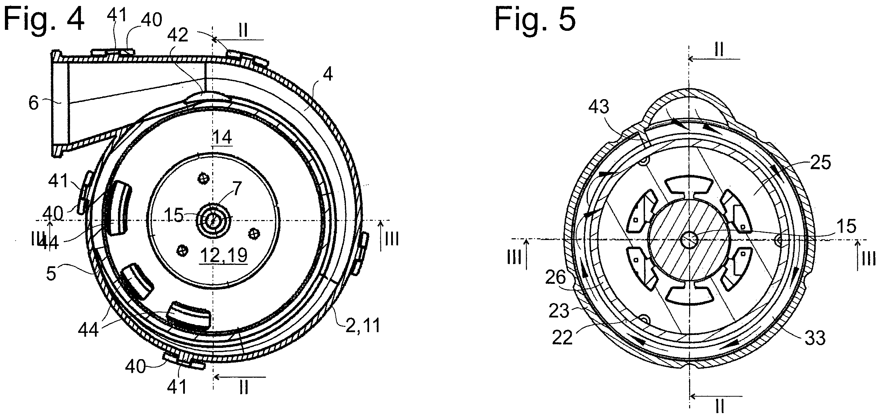

Die Schnittebene der Fig. 2 kreuzt den Blasluftkanal 4 kurz vor der der Ausblasöffnung 6. Zwischen der Wand 23 und der Zwischenwand 14 ist ein Einlass 42 ausgespart, durch den Luft aus dem Blasluftkanal abfließen und in den Kühlluftkanal 33 der Motorkammer 13 eindringen kann. Wie in Fig. 5 zu erkennen, ist benachbart zu dem Einlass 42 an der Wand 23 eine Trennwand 43 geformt, die sich radial nach innen bis zur Umfangsfläche 22 des Motors 12 erstreckt.

Die Schnittebene IV-IV der Fig. 4 liegt unterhalb des Schaufelrads 1 Wie insbesondere in dieser Fig. deutlich wird, ist in der Zwischenwand 14 an der dem Einlass 42 gegenüberliegenden Seite der Trennwand 43 ein Auslass 44, hier in Form von mehreren entlang des Randes der Zwischenwand 14 verteilten Öffnungen, gebildet. Die Trennwand 43 zwingt die über den Einlass 42 in den Kühlluftkanal 33 gelangte Luft, rings um den Motor 12 herum entgegengesetzt zur Drehrichtung des Schaufelrads 1 zum Auslass 44 zu fließen.

Der Auslass 44 mündet in die Radkammer 3, wie in Fig. 3 zu erkennen, unterhalb des Randes der Bodenplatte 18 und ist durch die Drehung der Bodenplatte 18 und durch den vom Schaufelrad 1 angetriebenen, in Höhe der Schaufeln 10 radial auf den Blasluftkanal 4 zulaufenden Luftstrom einem dynamischen Unterdruck ausgesetzt, der die Zirkulation durch den Kühlluftkanal 33 fördert.

Der Raum 34 ist durch den Dichtring 16 hermetisch von der Luftzirkulation im Kühlluftkanal 33 abgeschnitten, Gerüche, die von im Motor 12, insbesondere in einem an den Raum 34 angrenzenden Wälzlager 45, verwendetem Schmierstoff oder von stromführenden Teilen des Motors 12 im betriebswarmen Zustand abgegeben werden, können aus dem Raum 34 nicht entweichen.

Der Raum 35 ist zwar in dem hier gezeigten Ausführungsbeispiel nicht hermetisch von der Radkammer 3 abgeriegelt, dennoch gelangt auch von dort kein Geruch in den Blasluftstrom, da zwischen dem Raum 35 und einem benachbarten Wälzlager 46 die Welle 15 durch eine enge Öffnung des Deckels 27 verläuft, die keinen nennenswerten Luftaustausch zwischen dem Inneren des Motors 12 und dem Raum 35 zulässt.

Falls alternativ ein Motor verwendet wird, dessen dem Schaufelrad zugewandtes Wälzlager nicht wie gezeigt im Motorgehäuse abgeschirmt ist, kann die Öffnung der Zwischenwand 14, durch die sich die Welle 15 erstreckt, ähnlich der Öffnung des Deckels 27 so weit verengt werden, dass Geruchsstoffe aus dem Wälzlager 46 zwar in dem Raum 35 zwischen der Zwischenwand 14 und der Stirnfläche 19 verbreiten, von dort aber nicht in wahrnehmbarer Menge in die Radkammer 2 vordringen können.

**Bezugszeichen**

| | | | |
|---|---|---|---|
| 1 | Schaufelrad | 29 | Elektronikplatine |
| 2 | Gehäuse | 30 | Steckbuchse |
| 3 | Radkammer | 31 | Öffnung |
| 4 | Blasluftkanal | 32 | Kontaktstift |
| 5 | Anfangspunkt | 33 | Kühlluftkanal |
| 6 | Endpunkt/ Ausblas 0 öffnung | 34 | Raum |
| 7 | Drehachse | 35 | Raum |
| 8 | Oberteil | 36 | Bodenplatte |
| 9 | Ansaugöffnung | 37 | Flansch |
| 10 | Schaufel | 38 | gestufte Kontur |
| 11 | Unterteil | 39 | Schraube |
| 12 | Elektromotor | 40 | Rasthaken |
| 13 | Motorkammer | 41 | Rastnase |
| 14 | Zwischenwand | 42 | Einlass |
| 15 | Welle | 43 | Trennwand |
| 16 | Dichtring | 44 | Auslass |
| 17 | Dichtring | 45 | Wälzlager |
| 18 | Bodenplatte | 46 | Wälzlager |
| 19 | Stirnfläche | 47 | Stiftleiste |
| 20 | Stirnfläche | 48 | Stab |
| 21 | Hohlkehle | 49 | Rasthaken |
| 22 | Umfangsfläche | | |
| 23 | Wand | | |
| 24 | Aussparung | | |
| 25 | Statorpaket | | |
| 26 | Becher | | |
| 27 | Deckel | | |
| 28 | Schulter | | |

## Patentansprüche

1. Lüftereinheit mit einem Gehäuse (2), einem in einer Radkammer (3) des Gehäuses (2) untergebrachten Lüfterrad (1) und einem mit dem Lüfterrad (1) über eine Welle (15) verbundenen, in einer Motorkammer (13) des Gehäuses (2) untergebrachten Motor (12), wobei
der Motor (12) zwischen Wänden (14, 18) der Motorkammer (13) durch Puffer fixiert ist, wobei
wenigstens einer der Puffer ein Dichtring (16) ist, der die Welle (15) von einem sich in einem radialen Schnitt ringförmig um den Motor (12) erstreckenden Kühlluftkanal (33) trennt, **dadurch gekennzeichnet, dass**
der ringförmige Kühlluftkanal (33) an einer Stelle von einer Trennwand (43) unterbrochen ist und
ein Einlass (42) und ein Auslass (44) des Kühlluftkanals (33) benachbart zur Trennwand (43) auf verschiedenen Seiten derselben angeordnet sind.

2. Lüftereinheit nach Anspruch 1, **dadurch gekennzeichnet, dass** ein Innendurchmesser des Dichtrings (16) größer ist als ein Außendurchmesser eines Spalts zwischen gegeneinander rotierenden Teilen eines Lagers (45) der Welle (15).

3. Lüftereinheit nach Anspruch 1 oder 2, **dadurch gekennzeichnet, dass** die Wände (14, 18) in Längsrichtung der Welle (15) beabstandet sind.

4. Lüftereinheit nach Anspruch 1, 2 oder 3 **dadurch gekennzeichnet, dass** die Puffer zwei Dichtringe (16, 17) sind, und dass der Kühlluftkanal (33) durch die Dichtringe (16, 17), eine sich zwischen den Dichtringen (16, 17) erstreckende Außenfläche (22) des Motors und eine sich zwischen den Dichtringen (16, 17) erstreckende Wand (23) des Gehäuses (2) begrenzt ist.

5. Lüftereinheit nach Anspruch 4, **dadurch gekennzeichnet, dass** die Außenfläche (22) des Motors (12) durch ein Statorpaket oder durch eine an einem Statorpaket (25) dicht anliegende Wandung (26) gebildet ist.

6. Lüftereinheit nach Anspruch 1, **dadurch gekennzeichnet, dass** von Einlass (42) und Auslass (44) wenigstens einer mit der Radkammer (3) verbunden ist.

7. Lüftereinheit nach Anspruch 1 oder 6, **dadurch gekennzeichnet, dass** die Radkammer (3) einen sich rund um das Lüfterrad (1) erstreckenden Blasluftkanal (4) umfasst, und dass wenigstens der Einlass (42) des Kühlluftkanals (33) von dem Blasluftkanal (4) abzweigt.

8. Lüftereinheit nach Anspruch 7, **dadurch gekennzeichnet, dass** der Luftdurchsatz des Blasluftkanals (4) von einem inneren Ende (5) zu einem äußeren Ende (6) hin kontinuierlich zunimmt und dass von den Ein- und Auslässen (42, 44) der eine benachbart zum inneren Ende (5) und der andere benachbart zum äußeren Ende (6) von dem Blasluftkanal (4) abzweigt.

9. Lüftereinheit nach Anspruch 7 oder 8, **dadurch gekennzeichnet, dass** das Gehäuse (2) ein den Motor (12) aufnehmendes erstes Gehäuseteil (11) und ein zweites Gehäuseteil (8) mit einer Ansaugöffung (9) umfasst, die in einer Außenwand des Blasluftkanals (4) aneinandergrenzen.

10. Lüftereinheit nach Anspruch 9, **dadurch gekennzeichnet, dass** erstes und zweites Gehäuseteil (8, 11) miteinander durch Rasthaken (40) verbunden sind.

11. Lüftereinheit nach Anspruch 9 oder 10, **dadurch gekennzeichnet, dass** erstes und zweites Gehäuseteil (8, 11) reibschlüssig ineinandergreifende Konturen (38) aufweisen.

12. Lüftereinheit nach einem der Ansprüche 9 bis 11, **dadurch gekennzeichnet, dass** das Gehäuse (2) ferner eine Zwischenwand (14) zwischen Motor- und Radkammer (13, 3) umfasst, die am ersten Gehäuseteil (11) verschraubt ist.

13. Lüftereinheit nach einem der vorhergehenden Ansprüche, **dadurch gekennzeichnet, dass** eine Stiftleiste (47) wenigstens für die Stromversorgung des Motors (12) dicht in eine Wand (18) des Gehäuses (2) eingespritzt ist.

14. Gerät zur Atmungsunterstützung, **gekennzeichnet durch** eine Lüftereinheit nach einem der vorhergehenden Ansprüche.

## Claims

1. Blower unit having a housing (2), an impeller (1) accommodated in an impeller chamber (3) in the housing (2), and a motor (12), connected to the impeller (1) via a shaft (15), which is accommodated in a motor chamber (13) in the housing (2), wherein the motor (12) is fixed between walls (14, 18) of the motor chamber (13) by means of buffers, wherein at least one of the buffers being a sealing ring (16) which separates the shaft (15) from a passage for cooling air (33) which, in radial section, extends annularly around the motor (12), **characterized in that**
the annular passage for cooling air (33) is interrupted at one point by a dividing wall (43), and
an inlet (42) to and an outlet (44) from the passage for cooling air (33) are arranged adjacent the dividing wall (43) on different sides thereof.

2. Blower unit according to claim 1, **characterized in that** an inside diameter of the sealing ring (16) is larger than an outside diameter of a gap between parts of a bearing (45) on the shaft (15) which rotate against one another.

3. Blower unit according to claim 1 or 2, **characterized in that** the walls (14, 18) are spaced apart in the longitudinal direction of the shaft (15).

4. Blower unit according to claim 1, 2 or 3, **characterized in that** the buffers are two sealing rings (16, 17), and **in that** the passage for cooling air (33) is bounded by the sealing rings (16, 17), by an outer surface (22) of the motor which extends between the sealing rings 16, 17), and by a wall (23) of the housing (2) which extends between the sealing rings (16, 17).

5. Blower unit according to claim 4, **characterized in that** the outer surface (22) of the motor (12) is formed by a stator assembly or by a wall means (26) which rests tightly against a stator assembly (25).

6. Blower unit according to claim 1, **characterized in that** of the inlet (42) and outlet (44) at least one is connected to the impeller chamber (3).

7. Blower unit according to claim 1 or 6, **characterized in that** the impeller chamber (3) comprises a passage for blown air (4) which extends round the impeller (1), and **in that** at least the inlet (42) to the passage for cooling air (33) comes off the passage for blown air (4).

8. Blower unit according to claim 7, **characterized in that** the throughput of air through the passage for blown air (4) increases continuously from an inner end (5) to an outer end (6) and **in that**, of the inlet and outlet (42, 44), one comes off the passage for blown air (4) adjacent the inner end (5) and the other comes off it adjacent the outer end (6).

9. Blower unit according to claim 7 or 8, **characterized in that** the housing (2) comprises a first housing part (11) which receives the motor (12) and a second housing part (8) having an intake opening (9), which adjoin one another in an outer wall of the passage for blown air (4).

10. Blower unit according to claim 9, **characterized in that** the first and second housing parts (8, 11) are connected together by latching hooks (40).

11. Blower unit according to claim 9 or 10, **characterized in that** the first and second housing parts (8, 11) have outlines (38) which engage in one another frictionally.

12. Blower unit according to one of claims 9 to 11, **characterized in that** the housing (2) further comprises an intermediate wall (14) between the motor and impeller chambers (13, 3) which is screwed to the first housing part (11).

13. Blower unit according to one of the preceding claims, **characterized in that** a pin connector (47) at least for the supply of current to the motor (12) is injection moulded into a wall (18) of the housing (2) in such a way as to be sealed therein.

14. Apparatus for assisting respiration, **characterized by** a blower unit according to one of the preceding claims.

## Revendications

1. Ensemble ventilateur avec un carter (2), une roue de ventilateur (1) logée dans une chambre de roue (3) du carter (2) et un moteur (12) logé dans une chambre de moteur (13) du carter (2), relié à la roue de ventilateur (1) par le biais d'un arbre (15), dans lequel
le moteur (12) est fixé entre des parois (14, 18) de la chambre de moteur (13) par tampon, dans lequel
au moins un des tampons est une bague d'étanchéité (16), qui sépare l'arbre (15) d'un canal d'air de refroidissement (33) s'étendant dans une coupe radiale annulairement autour du moteur (12), **caractérisé en ce que**
le canal d'air de refroidissement annulaire (33) est interrompu à un endroit par une cloison (43) et
une entrée (42) et une sortie (44) du canal d'air de refroidissement (33) sont agencées de manière adjacente à la cloison (43) sur différents côtés de celle-ci.

2. Ensemble ventilateur selon la revendication 1, **caractérisé en ce qu'**un diamètre intérieur de la bague d'étanchéité (16) est supérieur à un diamètre extérieur d'une fente entre des pièces rotatives en sens opposé d'un palier (45) de l'arbre (15).

3. Ensemble ventilateur selon la revendication 1 ou 2, **caractérisé en ce que** les parois (14, 18) sont espacées dans le sens de la longueur de l'arbre (15).

4. Ensemble ventilateur selon la revendication 1, 2 ou 3, **caractérisé en ce que** les tampons sont deux bagues d'étanchéité (16, 17), et que le canal d'air de refroidissement (33) est délimité par les bagues d'étanchéité (16, 17), une surface extérieure (22) du moteur s'étendant entre les bagues d'étanchéité (16, 17) et une paroi (23) du carter (2) s'étendant entre les bagues d'étanchéité (16, 17).

5. Ensemble ventilateur selon la revendication 4, **caractérisé en ce que** la surface extérieure (22) du moteur (12) est formée par un empilage de tôles de stator ou par une paroi (26) s'appliquant de manière étanche contre un empilage de tôles de stator (25).

6. Ensemble ventilateur selon la revendication 1, **caractérisé en ce qu'**au moins une parmi l'entrée (42) et la sortie (44) est reliée à la chambre de roue (3).

7. Ensemble ventilateur selon la revendication 1 ou 6, **caractérisé en ce que** la chambre de roue (3) comprend un canal d'air de soufflage (4) s'étendant autour de la roue de ventilateur (1), et qu'au moins l'entrée (42) du canal d'air de refroidissement (33) bifurque du canal d'air de soufflage (4).

8. Ensemble ventilateur selon la revendication 7, **caractérisé en ce que** le débit d'air du canal d'air de soufflage (4) augmente continuellement d'une extrémité intérieure (5) vers une extrémité extérieure (6) et que parmi les entrées et sorties (42, 44) l'une bifurque de manière adjacente à l'extrémité intérieure (5) et l'autre bifurque de manière adjacente à l'extrémité extérieure (6) du canal d'air de soufflage (4).

9. Ensemble ventilateur selon la revendication 7 ou 8, **caractérisé en ce que** le carter (2) comprend une première partie de carter (11) recevant le moteur (12) et une deuxième partie de carter (8) avec une ouverture d'aspiration (9), qui sont contiguës dans une paroi extérieure du canal d'air de soufflage (4).

10. Ensemble ventilateur selon la revendication 9, **caractérisé en ce que** la première et deuxième partie de carter (8, 11) sont reliées l'une à l'autre par un crochet d'encliquetage (40).

11. Ensemble ventilateur selon la revendication 9 ou 10, **caractérisé en ce que** la première et deuxième partie de carter (8, 11) présentent des contours (38) s'imbriquant l'un dans l'autre par friction.

12. Ensemble ventilateur selon l'une quelconque des revendications 9 à 11, **caractérisé en ce que** le carter (2) comprend en outre une paroi intermédiaire (14) entre la chambre de moteur et de roue (13, 3), qui est vissée au niveau de la première partie de carter (11).

13. Ensemble ventilateur selon l'une quelconque des revendications précédentes, **caractérisé en ce qu'**une barrette mâle (47) est injectée au moins pour l'alimentation électrique du moteur (12) de manière étanche dans une paroi (18) du carter (2).

14. Appareil d'assistance respiratoire, **caractérisé par** un ensemble ventilateur selon l'une quelconque des revendications précédentes.
